# EUROPEAN PATENT SPECIFICATION

(11) **EP 2 282 810 B1**
(45) Date of publication and mention of the grant of the patent: **09.08.2017**
(21) Application number: 09759229.9
(22) Date of filing: 02.06.2009
(51) Int. Cl.: A61N 1/36, A61N 1/08, A61N 1/375, A61N 1/372

(54) **CONDUCTIVE COATING OF IMPLANTS WITH INDUCTIVE LINK**
LEITFÄHIGE BESCHICHTUNG VON IMPLANTATEN MIT INDUKTIVER VERBINDUNG
REVÊTEMENT CONDUCTEUR D'IMPLANTS AVEC LIAISON INDUCTIVE

(30) Priority: 03.06.2008 US 58319 P
(43) Date of publication of application: 16.02.2011
(73) Proprietor: Med-El Elektromedizinische Geräte GmbH, 6020 Innsbruck (AT)
(72) Inventor: ZIMMERLING, Martin, 6082 Patsch (AT)
(74) Representative: Downing, Michael Philip
(86) International application number: PCT/US2009/045950
(87) International publication number: WO 2009/149069

(56) References cited:
- WO-A-01/39830
- WO-A-01/97908
- WO-A-96/25832
- WO-A1-2005/061050
- WO-A2-2007/112044
- US-A- 4 991 582
- US-A- 5 527 348
- US-A1- 2006 009 806

## Description

This application claims priority from U.S. Provisional Patent Application 61/058,319, filed June 2008.

### Field of the Invention

The present invention relates to medical implants, and more specifically to a surface coating for such devices.

### Background Art

Some implantable devices such as Cochlear Implants (CI's) transfer electrical energy and data via an inductive link through the skin. This requires that the implanted receiving coils are not electrically shielded, which would interfere with the signal transfer. For that reason, implant coils are either encapsulated by a non-metallic housing (e.g. made of ceramics) or are embedded into silicone outside the hermetic encapsulation of the electronic circuit.

Just as with any surgical procedure, there is also some risk during implant surgery of postoperative infections at the surgical site. This risk is generally small and depends on several factors including hygiene standards in the operating room and surgical technique. One technical solution to further reduce the risk of bio-film growth and infection at the implant device is an antibiotic coating. One specific example would be a silver-based coating since silver ions are antibiotic (even against drug-resistant bacteria) and also prevent fungal decay around the implanted device. Depending on several factors (such as the silver concentration) a problem may arise in that silver coating over the inductive coil may cause some electrical shielding of the inductive link, thereby negatively affecting both power transfer to the implant device and also data communication in both directions. The inductive link may be influenced even if there is a high DC resistance of the conductive coating.

Implant devices also may have an internal magnet in the center of the implanted coil for providing an attractive magnetic force to a corresponding external magnet in the external coil. In some designs the internal magnet may be removable such as for magnetic resonance imaging (MRI) in order to avoid interactions between the internal magnet and the external MRI magnetic fields the attendant potential risks such as torque on the implant device, imaging artifacts and weakening of the internal magnet. A typical procedure is a first surgery to remove the internal magnet or to replace the magnet by a non-metallic space holder prior to MRI scanning, and then after the MRI scanning, a second surgery to replace the internal magnet.

Depending on the design of the removable magnet, there may be some dead space between the internal magnet and the surrounding part of the implant (e.g. a silicone material containing the implant coil). Such a dead space can potentially raise a risk of bio-film formation and associated infection which is difficult to treat.

Currently, various ways to avoid some of these problems include:
- No conductive coating in the area of the inductive coil
- Keep the conductive coating at a low level where the inductive link is not negatively affected
- For the internal magnet, to have no removable magnet or have a design (geometry) which keeps the dead space very small.

US 2006/0009806 of Medtronic Inc. discloses implantable medical devices with anti-infective agents disposed on the surface. WO 2005/061050 of Meditronic Inc., upon which the preamble of claim 1 is based, discloses an implantable medical device with an anti-infection agent impregnated in a housing. WO 2007/112044 also of Medtronic Inc. discloses an implantable medical device which has a portion of its housing coated with a metal.

### Summary of the Invention

The invention is defined in the independent claim 1. Preferred embodiments are described in the dependent claims. Embodiments of the present invention are direct to an implantable device that includes an implanted coil for receiving a transcutaneous coil signal from an external transmitting coil. A coil housing contains the coil and has a non-conductive surface. A conductive coating covers at least a portion of the housing surface and forms a non-shielding pattern that minimizes interaction with the coil signal.

In further specific embodiments, to claim 1, wherein the non-shielding pattern may form a web, mesh, and/or radial line pattern. The conductive coating may be an antibiotic coating and/or a silver-based coating.

The coil housing may be formed of a ceramic material and may also contain a signal processing module for processing the received coil signal. Embodiments may also have an electrode lead connected to the coil housing, wherein the conductive coating pattern further covers at least a portion of the electrode lead. The implantable device may be an element in a cochlear implant system.

Embodiments of the present invention also include an implantable device including an implanted magnet that interacts with an external magnet to maintain the external magnet in a constant position adjacent to the implanted magnet. A magnet housing contains the magnet. A therapeutic coating is between at least a portion of the magnet and the magnet housing for delivery of a therapeutic benefit in the vicinity of the therapeutic coating.

In further such embodiments, the therapeutic coating may specifically be an antibiotic coating and the therapeutic benefit may include an antibiotic effect. The therapeutic coating may be a silver-based coating and/or a colloidal-based coating, and the therapeutic benefit may include preventing formation of a bio-film in the vicinity of the therapeutic coating.

The implanted magnet may be a removable magnet. The magnet housing may be formed of a ceramic material and/or may further contain an implanted coil for receiving a transcutaneous coil signal from an external transmitting coil. The magnet housing also may include a signal processing module for processing the received coil signal. The implantable device may be an element in a cochlear implant system.

Embodiments of the present invention also include an implantable device having an implanted coil for receiving a transcutaneous coil signal from an external transmitting coil. A coil housing contains the implanted coil which is embedded in a non-shielding pattern of conductive containment material divided by non-conductive separating structures, and the pattern minimizes interaction of the containment material with the coil signal.

In specific such embodiments, the non-shielding pattern may form a web, mesh, or radial line pattern. The containment material may include an antibiotic component and/or a silver-based component and/or a colloidal-based component. The coil housing may be formed of a ceramic material and/or may also contain a signal processing module for processing the received coil signal. The implantable device may be an element in a cochlear implant system.

### Brief Description of the Drawings

Figure 1 shows an implantable device having a patterned conductive coating according to an embodiment of the present invention.
Figure 2 shows another type of implantable device having a patterned conductive coating according to another embodiment of the present invention.
Figure 3 shows an implantable device having inductive link coils embedded in a low conductivity structure according to an embodiment of the present invention.
Figure 4 A-B shows an implantable device having a removable magnet and using a therapeutic coating according to an embodiment of the present invention.
Figure 5 A-B shows another implantable device having a removable magnet and using a therapeutic coating according to another embodiment of the present invention.

### Detailed Description of Specific Embodiments

Embodiments of the present invention are directed to an implantable device that uses a surface coating and/or bulk material which are developed in a pattern that avoids many of the problems that arise in previous approaches. Some of the benefits which specific embodiments of a therapeutic surface coating may provide include, without limitation:
- unimpeded data and energy transfer through the inductively coupled transcutaneous link
- avoidance of RF-heating of the surface coating due to eddy currents (e.g. in the event of Magnetic Resonance Imaging (MRI) or even during normal use).
   ∘ This may be especially important during the charging phase of an implanted battery when a relatively high amount of RF power is sent over the inductive link.
- good back-telemetry data transfer properties.

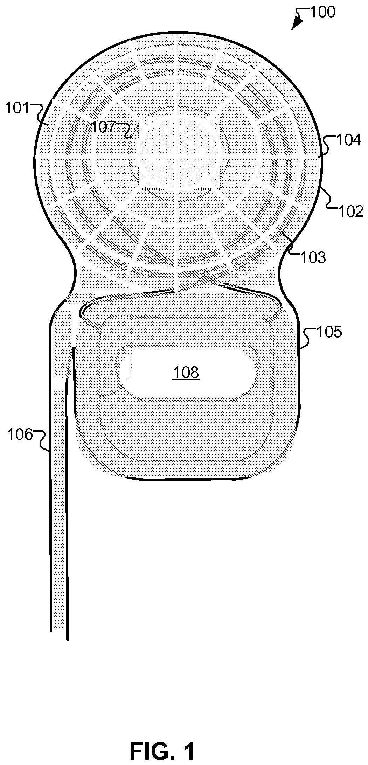

Figure 1 shows an implantable device **100** having a patterned conductive coating **101** according to an embodiment of the present invention. The upper circular portion is a coil housing **102** containing an implanted coil **103** for receiving a transcutaneous coil signal from an external transmitting coil. The coil housing **102** also contains an internal magnet **107** for maintaining an external magnet of an external transmitting coil in a constant position adjacent to the implanted magnet **107.**

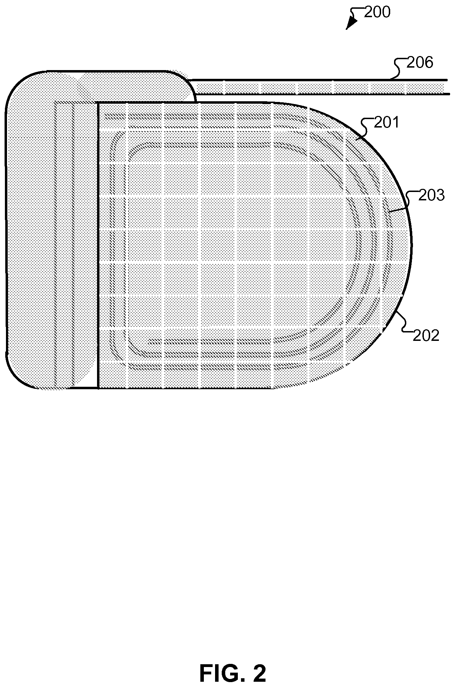

The coil housing **102** has a non-conductive outer surface **104,** at least a portion of which is covered by the conductive coating **101** which forms a non-shielding pattern that minimizes interaction with the coil signal. The conductive coating **101** does not homogeneously cover the complete surface area of the coil housing **102,** but rather is separated into smaller individual areas so that the negative influence on the inductive link is kept as small as possible, while at the same time, the area which is not coated shall be kept as small as possible so as to maximize the therapeutic benefits of the coating. For example, the non-shielding pattern of the conductive coating **101** may form a radial line pattern as shown in Fig. 1, or alternatively some other pattern such as a web or a mesh pattern (as in Fig. 2). The conductive coating **101** may be an antibiotic coating and/or a silver-based coating and/or a colloidal-based coating. Some embodiments may be limited by production processes and material properties (e.g. minimum effective thickness of non-conductive fragmentation lines) and efficacy of the conductive coating **101.** The relative amount percentage of the surface of the coil housing **102** (and the dimension of areas) not covered by the conductive coating **101** and/or the size of the non-conductive fragmentation paths should be minimized to preserve good therapeutic properties. There may be further benefits to the use of a conductive coating **101** beyond the therapeutic antibiotic effect mentioned. For example:
- to increase mechanical impact protection of the implantable device **100**
- to shield the implantable device **100** from ionizing radiation
It may further be useful to pattern the conductive coating **101** as discussed above for such considerations.

The implantable device **100** may also contain a signal processing module **105** for processing the received coil signal. For example, in a cochlear implant system, the signal processing module **105** contains circuitry for developing electrode stimulation signals which are output through an attached electrode lead **106,** the other end of which applies the stimulation signals to target nervous tissue. The conductive coating **101** may also cover some or all of the signal processing module **105** and/or the electrode lead **106** with or without the pattern used over the coil housing **102.** For example, with a relatively long electrode lead **106** there may be a risk of RF-induced heating of the conductive coating **101,** which can be mitigated by using a non-shielding (i.e. discontinuous or partitioned) pattern. There may be no conductive coating **101** over some elements of the implantable device **100** such as, for example, electrode ground contact **108.**

Figure 2 shows an example of another type of implantable device **200** having a mesh-patterned conductive coating **201** according to another embodiment of the present invention. In this embodiment, a single implant housing **202** made of a non-conductive ceramic material which contains the implanted coil **203** as well as the internal magnet and signal processing module (not shown). In this embodiment, the conductive coating **201** covers the entire implantable device **200** with the pattern extending over the implanted coil **203** and the electrode lead **206,** with the remainder of the coating being unpatterned.

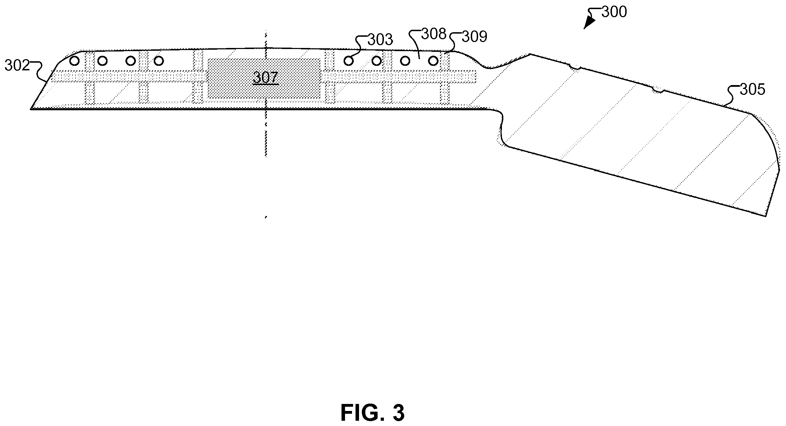

Figure 3 shows a cross-sectional view of an implantable device **300** similar to the two-part device in Fig. 1, having a coil housing **302** and a separate signal processing module **305.** Within the coil housing **302** are inductive link coils **303** for receiving a transcutaneous coil signal from an external transmitting coil. The coil housing **302** also contains an implanted magnet **307** that interacts with an external magnet to maintain the external magnet in a constant position adjacent to the implanted magnet.

The inductive link coils **303** are embedded in a low conductivity structure arranged in a non-shielding pattern of conductive containment material **308** (e.g., silicone impregnated with conductive material) which is divided by non-conductive separating structures **309,** where the pattern minimizes interaction of the containment material **308** with the coil signal. In specific such embodiments, the non-shielding pattern may form a web, mesh, or radial line pattern. In the embodiment shown in Fig. 3, the non-conductive separating structures **309** separate individual link coils **303** from each other to minimize the shielding effect of the surrounding conductive containment material **308.** The containment material **308** may include an antibiotic component and/or a silver-based component. It may be beneficial to implement non-conductive fragmentations need across the complete cross-section of the inductive link coils **303.**

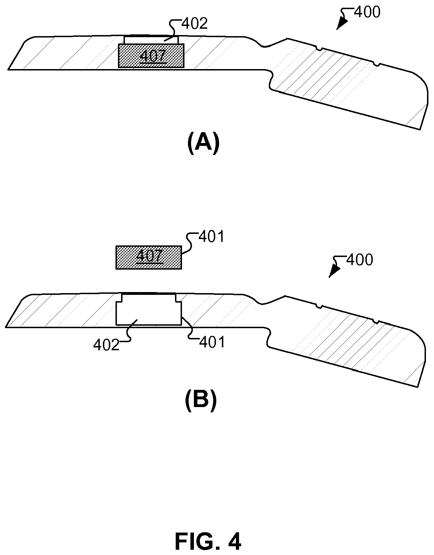

Figure 4 A-B shows an implantable device **400** having a removable internal magnet **407** and using a therapeutic coating according to an embodiment of the present invention. The cylindrical internal magnet **407** is contained in a corresponding cylindrical magnet housing **402** and interacts with an external magnet to maintain the external magnet in a constant position adjacent to the implanted magnet **407.** In one specific embodiment, the magnet housing **402** is in the form of a pocket of soft silicone material having an opening at the top through which the internal magnet **407** may be surgically removed when needed.

A therapeutic coating **401** covers the external surface of the implanted magnet **407** and the corresponding surfaces of the magnet housing **402** which engage the internal magnet **407.** The therapeutic coating **401** provides of a therapeutic benefit such as preventing formation of a bio-film in the vicinity of the therapeutic coating, thereby avoiding infection. Specifically, the therapeutic coating **401** may include antibiotic coating and/or a silver-based coating. It may also be useful to provide a therapeutic coating **401** on any dummy parts (e.g., a non-metallic space holder replacing the internal magnet **407** during an MRI) and/or replacement magnets (inserted after the MRI).

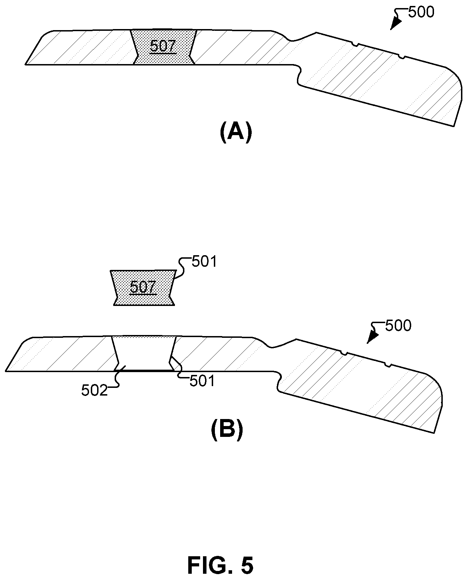

As with the conductive coatings discussed above, the therapeutic coating **401** may also be arranged in a non-uniform pattern. Figure 5 A-B shows another implantable device **500** having a different shaped non-cylindrical removable internal magnet **507** and using a therapeutic coating **501** according to another embodiment of the present invention. In some specific embodiments, it may also be useful to physically seal the dead space between the magnet and the magnet housing and/or provide a tight fit between them that prevents micro-movements of the magnet relative to the magnet housing when the external coil is removed or placed over the implant, in order to further reduce the risk of bio-film growth in the magnet area.

Although various exemplary embodiments of the invention have been disclosed, it should be apparent to those skilled in the art that various changes and modifications can be made which will achieve some of the advantages of the invention without departing from the scope of the appended claims.

## Claims

1. An implantable device (100) comprising an implantable coil (103) for receiving a transcutaneous coil signal from an external transmitting coil, wherein the implantable device
a coil housing (102) containing the coil (103) having a non-conductive surface (104);
an electrode lead connected to the housing (102); and
a conductive coating (101) containing therapeutic material and covering at least a portion of the outer surface of the housing and at least a portion of the electrode lead
**characterized in that** the conductive coating (101) is formed in a non-shielding pattern selected from a web pattern, a mesh pattern or a radial line pattern, that minimizes interaction with the coil signal.

2. An implantable device (100) according to claim 1, wherein the therapeutic material includes an antibiotic component.

3. An implantable device (100) according to claim 1, wherein the therapeutic material includes a silver-based component.

4. An implantable device (100) according to claim 1, wherein the therapeutic material includes a colloidal-based component.

5. An implantable device (100) according to claim 1, wherein the coil housing (102) is formed of a ceramic material.

6. An implantable device (100) according to claim 1, wherein the coil housing (102) further contains a signal processing module (105) for processing the received coil signal.

7. An implantable device (100) according to claim 1, wherein the implantable device (100) is an element in a cochlear implant system.

## Patentansprüche

1. Implantierbare Vorrichtung (100), umfassend eine implantierbare Spule (103) zum Empfangen eines transkutanen Spulensignals von einer externen Senderspule, wobei die implantierbare Vorrichtung umfasst:
ein Spulengehäuse (102), das die Spule (103) enthält, mit einer nicht leitfähigen Oberfläche (104);
eine mit dem Gehäuse (102) verbundene Elektrodenleitung; und
eine leitfähige Beschichtung (101), die therapeutisches Material enthält und wenigstens einen Teil der Außenoberfläche des Gehäuses und wenigstens einen Teil der Elektrodenleitung bedeckt;
**dadurch gekennzeichnet, dass** die leitfähige Beschichtung (101) in einer nicht abschirmenden Struktur ausgewählt aus einer Netzstruktur, einer Siebstruktur oder einer Radiallinienstruktur, die die Wechselwirkung mit dem Spulensignal minimiert, gebildet ist.

2. Implantierbare Vorrichtung (100) gemäß Anspruch 1, wobei das therapeutische Material eine antibiotische Komponente enthält.

3. Implantierbare Vorrichtung (100) gemäß Anspruch 1, wobei das therapeutische Material eine Komponente auf Silberbasis enthält.

4. Implantierbare Vorrichtung (100) gemäß Anspruch 1, wobei das therapeutische Material eine Komponente auf Kolloidbasis enthält.

5. Implantierbare Vorrichtung (100) gemäß Anspruch 1, wobei das Spulengehäuse (102) aus einem Keramikmaterial besteht.

6. Implantierbare Vorrichtung (100) gemäß Anspruch 1, wobei das Spulengehäuse (102) ferner ein Signalverarbeitungsmodul (105) zum Verarbeiten des empfangenen Spulensignals enthält.

7. Implantierbare Vorrichtung (100) gemäß Anspruch 1, wobei die implantierbare Vorrichtung (100) ein Element in einem Cochleaimplantatsystem ist.

## Revendications

1. Dispositif implantable (100) comprenant une bobine implantable (103) pour recevoir un signal transcutané de bobine en provenance d'une bobine d'émission externe, dans lequel le dispositif implantable comprend :
un boîtier de bobine (102) contenant la bobine (103) comportant une surface non conductrice (104) ;
un fil d'électrode raccordé au boîtier (102) ; et
un revêtement conducteur (101) contenant une substance thérapeutique et recouvrant au moins une partie de la surface externe du boîtier et au moins une partie du fil d'électrode ;
**caractérisé en ce que** le revêtement conducteur (101) se présente sous la forme d'un motif non protecteur sélectionné parmi un motif de tissu, un motif maillé ou un motif de ligne radiale qui réduit à un minimum une interaction avec le signal de bobine.

2. Dispositif implantable (100) selon la revendication 1, dans lequel la substance thérapeutique comprend un composant antibiotique.

3. Dispositif implantable (100) selon la revendication 1, dans lequel la substance thérapeutique comprend un composant à base d'argent.

4. Dispositif implantable (100) selon la revendication 1, dans lequel la substance thérapeutique comprend un composant colloïdal.

5. Dispositif implantable (100) selon la revendication 1, dans lequel le boîtier de bobine (102) est composé d'un matériau céramique.

6. Dispositif implantable (100) selon la revendication 1, dans lequel le boîtier de bobine (102) contient en outre un module de traitement de signal (105) pour traiter le signal de bobine reçu.

7. Dispositif implantable (100) selon la revendication 1, dans lequel le dispositif implantable (100) est un élément dans un système d'implant cochléaire.
